Europäisches Patentamt

European Patent Office

Office européen des brevets

(19)

(11) Publication number: **0 451 979 A2**

# EUROPEAN PATENT APPLICATION

(12)

(21) Application number: 91302533.4

(22) Date of filing: 22.03.91

(51) Int. Cl.$^5$: **C07C 209/48**

(30) Priority: 10.04.90 US 506747

(43) Date of publication of application:
16.10.91 Bulletin 91/42

(84) Designated Contracting States:
DE FR GB

(71) Applicant: TEXACO CHEMICAL COMPANY
3040 Post Oak Boulevard
Houston, Texas 77056 (US)

(72) Inventor: Zimmerman, Robert Leroy
4202 Cordova
Austin, Texas 78759 (US)

(74) Representative: Brock, Peter William et al
URQUHART-DYKES & LORD 91 Wimpole
Street
London W1M 8AH (GB)

(54) **Continuous preparation of secondary amines from nitriles using cobalt and zirconium.**

(57) Secondary amines can be produced from nitriles (such as tallow nitrile) in high selectivity by using cobalt promoted by zirconium as catalyst. Reaction is advantageously carried out continuously in two stages, using hydrogen and added ammonia in a first stage, and hydrogen alone without added ammonia in a second stage.

EP 0 451 979 A2

The invention relates to the production of secondary amines from nitriles, and, in one aspect, more particularly relates to the continuous production of secondary amines from nitriles using a single transition metal catalyst.

It has long been known that nitriles can be reduced to give amines. Typically a mixture of primary, secondary and tertiary amines is produced, and a common aim is to devise a process whose result would be a high yield of only one of the possible products; that is, high selectivity to a particular product. The reaction is understood to proceed in two stages, and often the process is carried out as two separate steps, often using a different catalyst for each step. Frequently, the reaction is run as a batch reaction, inasmuch as good selectivities have been difficult to achieve using continuous processes.

Particularly useful products from the reaction are the secondary amines. They have found such widespread uses as textile additives, disinfectants, antistatic agents, and use for the production of organophilic ammonium bentonites. Especially useful are the unsaturated long-chain aliphatic secondary amines, since the corresponding quaternary ammonium salts can provide softness and antistaticity to various fabrics and hair, and can also be used as a softener for treating fabrics to provide water absorbency and handling ease. The secondary amine ditallowamine is useful in the preparation of surfactants, but has never been continuously prepared in high selectivity from tallow nitrile. Tallow nitrile has sixteen to eighteen carbon atoms ($C_{16}$ to $C_{18}$).

Of particular interest is GB-A-1180972, which teaches that aliphatic, saturated and unsaturated secondary fatty amines can be prepared by passing the corresponding fatty acid nitrile at 140-200°C and 3.04 to 20.27 MPa (30-200 atm) of hydrogen, together with water, over a solid catalyst consisting of 20% copper, 0.8% chromium and 1% alkali metal, with a wide-pored silica gel having a specific area of 250-350 m.$^2$/g as the support. GB-A-1323351 describes a process for making aliphatic saturated secondary amines from nitriles having 8 to 22 carbon atoms, where the starting material is hydrogenated in a first step to yield a mixture of saturated amines, and this mixture is continuously desaminated (i.e. ammonia is split off) in a second step, optionally with the addition of hydrogen, each step being carried out in the presence of a fixed bed of hydrogenation catalyst. The first step is conducted at a hydrogen pressure of 10.2 to 30.5 MPa (100 to 300 atmospheres gauge) and at a temperature of 100 to 200°C, while the second step is conducted at a pressure from 0 to 51 MPa (0 to 50 atmospheres) and at a temperature of 120 to 220°C. The catalysts used are cobalt in the first step and then copper; or alternatively nickel in the first step and cobalt in the second.

Of lesser importance is GB-A-759291, which teaches production of secondary aliphatic hydrocarbon amines via a batch reaction using a nickel hydrogenation catalyst. A similar process is described in US-A-2811556, except that a copper oxide/chromium oxide catalyst is used.

JP-A-6200-901, teaches the preparation of long-chain secondary amines by the reduction of aliphatic nitriles having 8 to 22 carbon atoms over nickel catalysts at 0.1 to 0.7 MPa (0-6 kg/cm$^2$ gauge) and 200 to 230°C while removing more than 85% formed $NH_3$. Thus, 250 g. of tallow nitrile was reduced over 0.5 g. of Ni catalyst at 200-300°C and 0.5 MPa (5kg/cm$^2$), while removing 93% formed $NH_3$ to give 240 g. of a mixture of primary (3.1%), secondary (91.1%) and tertiary amine (4.3%) amines.

EP-B-0021162 teaches the production of alkylamines with 12 to 22 carbon atoms by hydrogenating corresponding fatty nitriles in the presence of a nickel or cobalt catalyst. The hydrogen gas reactant is recirculated after removal of ammonia. The new feature is that throughout the reaction the water content of the circulating gas is adjusted to not above 5 g. per cubic meter, under practically zero-pressure conditions, before recycle. Additionally, a process for selectively preparing an unsaturated long-chain aliphatic secondary amine at a high yield involving reducing an unsaturated aliphatic nitrile having 8 to 22 carbon atoms, or a nitrile mixture containing said nitrile, with hydrogen in the presence of a nickel hydrogenation catalyst and a carboxylic acid amide at a reaction temperature of 160 to 200°C, is disclosed in EP-A-0232097.

A process for the selective production of aliphatic secondary amines from primary amines from $C_8$ $_{-22}$ primary amines using dehydrogenation/hydrogenation catalysts is briefly mentioned in the English abstract to DD-A-133229. In a first stage, the primary amine is dehydrogenated at normal pressure and at 170 to 260°C, by treatment with a inert gas ($N_2$) in an amount of 5 to 150 l/mol./h, for 30 to 60 minutes until a degree of conversion of the starting material of 85-98% is achieved. The resulting dehydrogenated product is then reacted with hydrogen at 100 to 140°C and 0.1 to 5.1 MPa (0 to 50 atmospheres) for 10 to 30 minutes to form the secondary amine. The nature of the catalyst was not mentioned in the abstract.

EP-A-0384542, which is a document falling within the state of the art according to Article 54(3) EPC, discloses a process for preparing secondary alkylamines by selective hydrogenation of alkyl nitriles, using a catalyst comprising nickel promoted by copper, optionally with cobalt as a co-promoter.

The present invention is concerned with the problem of providing a continuous process for producing fatty secondary amines, simply and with high selectivity, making use of only one catalyst.

The present invention provides a continuous process for the preparation of secondary amines which comprises continuously passing a nitrile over a catalyst comprising cobalt promoted with zirconium.

In a preferred embodiment the process is carried out in two stages, in the first of which the nitrile is passed over the catalyst, in the presence of added ammonia and hydrogen to produce an intermediate reaction product; while in the second stage, the intermediate reaction product is passed over the catalyst in the presence of hydrogen but the absence of ammonia.

It has been discovered that secondary amines, particularly fatty secondary amines such as ditallowamine, may be produced in high selectivity by passing the corresponding nitrile, such as tallow nitrile, over a cobalt catalyst promoted with an effective amount of zirconium.

The invention is particularly suited for producing fatty secondary amines from fatty nitriles, which are defined as having from 8 to 22 carbon atoms. A preferred feedstock, because it is relatively inexpensive, is tallow nitrile, which has 16 to 18 carbon atoms. Of course, the resulting secondary amine has twice as many carbon atoms as the nitrile used as starting material.

As noted, the catalyst should be a cobalt hydrogenation-dehydrogenation catalyst, such as cobalt oxide. Another suitable cobalt catalyst is cobalt metal, but the process is not limited to these two cobalt materials.

The promoter is zirconium, for example in the form of zirconium oxide. In one embodiment, the proportion of cobalt in the catalyst should be from 30 to 70%, preferably from 40 to 60%. The amount of zirconium promoter, e.g. in oxide form, may be from 1 to 5%, more preferably from 2 to 3%.

The catalyst may be supported upon kieselguhr, also known as diatomaceous earth, diatomite or infusorial earth. The catalyst may also be supported on materials including, but not limited to alumina, silica or titania. The reaction may be conducted in two steps. Surprisingly, it was discovered that the same catalyst may be used even in a two-step process. The inventive process also gives product having a higher secondary amine content than other known continuous processes.

The reaction is preferably conducted at elevated temperatures and pressures. For example, the temperature may be from 100 to 200°C, preferably from 130 to 180°C. The pressure may be from 0.44 to 34.57 MPa (50 to 5000 psig), and more preferably from 1.48 to 6.99 MPa (200 to 1000 psig). It is preferred that ammonia and hydrogen are present during the reaction. If the reaction is conducted in two continuous stages, ammonia is preferably not present in the second stage. The absence of ammonia in the second step is preferred, since primary amines are formed in the first reaction, and the production of the secondary amines from two primary amines in the second step also produces an ammonia molecule. Thus, the absence of ammonia in the second step would facilitate the second reaction.

The use of a continuous reaction has advantages over the batch reactions in that no filtration or loss of catalyst is experienced, since a fixed bed is used in the continuous reaction. The invention will be illustrated in greater detail with reference to the following Examples.

## Examples 1-2

### Production of Ditallowamine

Example 1. For the following experiments a tubular reactor filled with 430 cc of catalyst was used. The catalyst comprised 52% of cobalt oxide and 2.5% of zirconium oxide on a kieselguhr support. Tallow nitrile was fed at a rate of 313g/h (0.69 lb/h) ammonia at 54.5g/h (0.12 lb/h) and hydrogen at 355 l/h. The reactor pressure was 3.55 MPa (500 psig) and the hot spot temperature was 145°C. As the effluent came out of the reactor, the excess ammonia was allowed to evaporate. This product contained 36% of primary amine, 62% of secondary amine and 2% of tertiary amine.

Example 2 The product from Example 1 was then run through the reactor again at a rate of 340.5 g/h (0.75 lb/h). No ammonia was added to the reactor. The hydrogen feed rate was 355 l/h and the pressure was still 3.55 MPa (500 psig). The temperature was 150°C.

The crude product was stripped under vacuum to remove ammonia. The final product contained 2% of primary amine, 93% of secondary amine and 5% of tertiary amine. The yield of secondary amine (hydrogenated ditallowamine) is better than the yields of 90.3% in Example 1 of GB-A-1323351 or 90.5% in Example 1 of GB-A-1180972.

Such excellent results in the continuous production of ditallowamine from tallow nitrile using a single catalyst are unknown in the art.

Example 3 To illustrate the beneficial effect of employing ammonia during the first stage, but not in the second stage, a series of runs A to H was carried out, using the zirconium-promoted cobalt catalyst. The same total throughput of tallow nitrile and ammonia (367g/h//0.81 lb/h) was employed in each run. Table 1 shows the effect of including ammonia in the first stage, while Table 2 shows the effect of excluding added ammonia in the second stage.

3

## Table 1

| | | ---Feed reactants--- | | | ----Products----- | |
| Run | Temp. °C | Tallow nitrile g/h | NH₃ g/h | Primary amine, % | Secondary amine, % | Tertiary amine, % |
|---|---|---|---|---|---|---|
| A | 160 | 367 | – | 41 | 51 | 7 |
| B | 180 | 367 | – | 20 | 71 | 9 |
| C | 180 | 340 | 27 | 44 | 51 | 5 |
| D | 180 | 313 | 54 | 21 | 77 | 3 |

## Table 2

| | | ---Feed reactants--- | | | ---Products--- | |
| Run | Temp °C | Tallow nitrile g/h | NH₃ g/h | Primary amine, % | Secondary amine, % | Tertiary amine, % |
|---|---|---|---|---|---|---|
| E | 150 | 340 | 27 | 28 | 71 | 1 |
| F | 170 | 340 | 27 | 12 | 84 | 5 |
| G | 150 | 340 | – | 21 | 78 | 1 |
| H | 160 | 340 | – | 8 | 88 | 3 |

It will be seen from Table 1 that Runs A and B, using no ammonia produced an amount of tertiary amine which is too high to provide an acceptable product. Comparison of Runs A and C, providing the same proportion of secondary amine, shows that less of the undesirable tertiary amine is obtained in the presence of ammonia. Runs B, C and D, all conducted at the same temperature show that an increased amount of ammonia provides a reduced amount of tertiary amine.

In Table 2, the feed used in the second stage comprised 70% of primary amine, 29% of secondary amine and 0.1% of tertiary amine. Runs G and H, using no added ammonia, resulted in products having a higher proportion of secondary amine and a lower proportion of tertiary amine than the comparable Runs E and F where ammonia was present.

The results set out in Tables 1 and 2 demonstrate that best results are obtained when ammonia is added in the first stage, and excluded in the second stage.

Example 4 (for Comparison)

This Example compares the result of using different catalysts which are not according to the invention. The results set out in Table 3 show the results achieved in four comparative Runs I, J, K and L, compared with those achieved in Example 1. The conditions are identical with those used in Example 1, except for the different catalysts and Temperatures indicated in Table 3.

## Table 3

| Run | Catalyst | Temp. °C | Primary amine % | Secondary amine % | Tertiary amine % |
|---|---|---|---|---|---|
| I | Co on Silica | 180 | 21 | 67 | 12 |
| J | Co on Silica | 170 | 28 | 65 | 7 |
| K | Co-Cu-Cr | 180 | 27 | 64 | 9 |
| L | Co-Cu-Cr | 170 | 33 | 62 | 5 |
| Example 1 | Co/Zr | 145 | 36 | 62 | 2 |

It will be seen that Example 1, using cobalt promoted with zirconium gave 36% of primary amine, 62% of secondary amine and 2% of tertiary amine. None of Runs I-L give results with this low content of tertiary amine. While the primary amine may be converted into the secondary amine in the subsequent second pass, tertiary amine cannot be transformed into secondary amine.

While the comparative catalysts used in Table 3 (Co on silica; Co-Cu-Cr) gave higher tertiary amine contents at about the same secondary amine content, the tertiary amines cannot be converted further to give a higher secondary amine that contains a low tertiary amine content.

4

**Claims**

1. A continuous process for the preparation of secondary amines which comprises continuously passing a nitrile over a catalyst comprising cobalt promoted with zirconium.

2. A process according to Claim 1 characterized in that the catalyst is supported on kieselguhr.

3. A process according to Claim 1 or 2 characterized in that the temperature is from 100 to 200°C and the pressure is from 0.44 to 34.57 MPa (50 to 5000 psig).

4. A process according to any one of Claims 1 to 3 characterized in that the catalyst comprises cobalt oxide and zirconium oxide.

5. A process according to Claim 4 characterized in that the catalyst comprises at least 30% of cobalt oxide and at least 1% of zirconium oxide.

6. A process according to any one of Claims 1 to 5 characterized in that the nitrile has from 8 to 22 carbon atoms.

7. A process according to any one of Claims 1 to 6 characterized in that the nitrile is contacted with the catalyst in the presence of ammonia and hydrogen.

8. A process according to any one of Claims 1 to 6 characterized in that in a first stage, the nitrile is passed over the catalyst, in the presence of added ammonia and hydrogen to produce an intermediate reaction product; and in a second stage, the intermediate reaction product is passed over the catalyst in the presence of hydrogen but the absence of ammonia.